# EUROPÄISCHE PATENTSCHRIFT

(11) **EP 2 296 677 B1**
(45) Veröffentlichungstag und Bekanntmachung des Hinweises auf die Patenterteilung: **02.11.2011**
(21) Anmeldenummer: 09749319.1
(22) Anmeldetag: 20.05.2009
(51) Int. Cl.: A61K 35/74, A61P 1/12

(54) **PROBIOTISCHER FUTTERMITTEL- UND/ODER TRINKWASSERZUSATZ UND VERWENDUNG**
PRO BIOTIC FOODSTUFF AND/OR DRINKING WATER ADJUNCT AND USE THEREOF
ALIMENT POUR ANIMAUX ET/OU ADDITIF D'EAU POTABLE PROBIOTIQUE(S) ET UTILISATION

(30) Priorität: 23.05.2008 AT 2962008 U
(43) Veröffentlichungstag der Anmeldung: 23.03.2011
(73) Patentinhaber: Erber Aktiengesellschaft, 3130 Herzogenburg (AT)
(72) Erfinder: KLOSE, Vivane, A-1210 Wien (AT); SCHATZMAYR, Gerd, A-3430 Tulln (AT); BINDER, Eva, Maria, A-3430 Tulln (AT); HENIKL, Sabine, A-3213 Frankenfels (AT)
(74) Vertreter: Cunow, Gerda
(86) Internationale Anmeldenummer: PCT/AT2009/000209
(87) Internationale Veröffentlichungsnummer: WO 2009/140708

(56) Entgegenhaltungen:
- WO-A-2006/133472

## Beschreibung

Die vorliegende Erfindung bezieht sich auf einen probiotischen Futtermittel- und/oder Trinkwasserzusatz enthaltend wenigstens einen Mikroorganismus zur kompetitiven Hemmung von pathogenen Keimen des Stammes Brachyspira hyodysenteriae im Verdauungstrakt von Tieren, sowie auf eine Verwendung derselben.

Futtermittel- und/oder Trinkwasserzusätze, welche einen Mikroorganismus oder auch Mischungen von Mikroorganismen enthalten, werden verstärkt sowohl im humanen als auch tierischen Anwendungsbereich eingesetzt, um Infektionen durch pathogene Keime, wie beispielsweise Salmonellen oder E. coli, Campylobacter, Clostridien etc., möglichst hintanzuhalten. Der Einsatz von derartigen Mischungen von Mikroorganismen beruht auf der sogenannten "competitive exclusion" (CE), bei welcher versucht wird, mit sogenannten "guten" Bakterien die pathogenen oder krankheitserregenden Bakterien, d.h. gesundheitsschädigende bzw. -beeinträchtigende Bakterien, zu verdrängen bzw. auszuschließen. Hiebei hemmt das Wachstum der guten Bakterien jenes der pathogenen oder krankheitserregenden Bakterien, beispielsweise indem sie im Milieu des Verdauungstrakts einen Wachstumsvorteil haben und dadurch schneller proliferieren. Konkret wird dabei ein spezielles Bakterium bzw. eine spezielle Mischung von Bakterien eingesetzt, welche auch aus dem Verdauungstrakt isoliert werden kann, wobei versucht wird, entweder möglichst breite Spektren an Darmbakterien oder sehr spezifische Darmbakterien zu verabreichen, um ein gewünschtes Anwendungsgebiet zu erzielen. Es hat sich jedoch der Einsatz von einem möglichst breiten Spektrum von Darmbakterien, welche

nicht spezifiziert sind, als problematisch erwiesen, da einerseits etwaige Probleme undefinierter Mischungen, wie beispielsweise die Übertragung von Antibiotikaresistenzen oder Krankheiten, kaum vermieden werden können, und andererseits auch die Verabreichung von Undefinierten Mischungen durch regionale oder internationale Regulierungen und Gesetzgebungen eingeschränkt ist, um nicht unerwartete bzw. unerwünschte Ergebnisse durch die Verabreichung derartiger Mischungen zu erzielen. Es wird daher auf den Einsatz definierter Mischungen, insbesondere auf definierte Probiotikakulturen, die aus einem oder mehreren Stämmen bestehen, zurückgegriffen werden müssen, um unerwartete Wirkungen zu vermeiden und der Gesetzgebung Genüge zu tun.

Aus der AT-B 501 919 bzw. der WO 2006/133472 A ist bereits ein probiotischer, gesundheits- bzw. leistungsfördernder Futtermittel- und/oder Trinkwasserzusatz für Tiere bekannt geworden, welcher eine Mischung aus Mikroorganismen enthält und welcher Futtermittel- und/oder Trinkwasserzusatz im Verdauungssystem von Säugern und/oder Nutzvögeln dahingehend wirkt, daß die schädliche Wirkung von unerwünschten Keimen, wie Salmonellen, E. coli Clostridien und dgl. verhindert bzw. zumindest reduziert wird.

Ein besonderes Augenmerk wird neuerdings auf die schweinepathogenen Keime Brachyspira hyodysenteriae gelegt. Diese schweinepathogenen Keime gehören zu den Spirochaeten und spielen in der Tierproduktion eine bedeutende Rolle als Erreger der Schweinedysenterie. Dysenterie zählt in Schweinemastbeständen weltweit zu den häufigsten und verlustreichsten infektiösen Erkrankungen und tritt in einem Schweinebestand in der Regel als schleichende Infektion mit Diarrhöe in Erscheinung. Die Krankheitshäufigkeit bei der Schweinedysenterie beträgt ca. 90 % und die Sterblichkeit kann bis zu 30 % betragen. Ursachen für die Häufigkeit der Erkrankung sind nicht nur die Tatsache, daß die Krankheitserreger, die mit dem Kot ausgeschieden werden, in der Folge von den Ferkeln wieder oral aufgenommen werden, sondern auch der Streß, dem Jungtiere sehr häufig beispielsweise beim Umstallen oder Absetzen ausgesetzt sind. Im Zuge von experimentellen Untersuchungen wurde herausgefunden, daß es sich bei der Schweinedysenterie um eine synergistische Infektion zwischen Brachyspira hyodysenteriae und anderen anaeroben Erregern, wie beispielsweise Fusobacterium necrophorum, Clostridium perfringens oder aeroben bzw. mikroaerophilen Erregern, wie E. coli, Campylobacteri jejuni usw., handelt. Da jedoch Brachyspira hyodysenteriae für die Entstehung der sichtbaren Schäden im Darm verantwortlich ist, gilt dieser Keim als Haupterreger. Indem der Erreger auch nach einer Behandlung und überstandener Krankheit im Darm verbleiben kann bzw. persistieren kann, kann es dadurch zu schweren Verlusten in einem Zuchtbetrieb nicht nur durch Verenden kranker Tiere, sondern auch durch deutlich verminderte Tagesgewichtszunahmen, verlängerte Mastdauer und den Einsatz von Antibiotika kommen.

Die vorliegende Erfindung zielt nun darauf ab, einen probiotischen, Gesundheit bzw. Leistung fördernden Futtermittel- und/ oder Trinkwasserzusatz für Tiere zur Verfügung zu stellen, welcher pathogene Keime des Stammes Brachyspira hyodysenteriae kompetitiv im Verdauungstrakt von Tieren hemmt, wodurch eine Leistungssteigerung in Zucht- bzw. Mastbetrieben erzielt werden kann.

Zur Lösung dieser Aufgaben ist der probiotische Futtermittel- und/oder Trinkwasserzusatz, enthaltend wenigstens einen Mikroorganismus zur kompetitiven Hemmung von pathogenen Keimen des Stammes Brachyspira hyodysenteriae im Verdauungstrakt von Tieren, im wesentlichen dadurch gekennzeichnet, daß der wenigstens eine Mikroorganismus aus der Gruppe Bifidobacterium thermophilium DSMZ 19765, Lactobacillus salivarius DSMZ 21286, Lactobacillus sobrius DSMZ 21285 gewählt ist. Dadurch, daß der wenigstens eine Mikroorganismus aus der Gruppe Bifidobacterium thermophilium DSMZ 19765, Lactobacillus salivarius DSMZ 21286, Lactobacillus sobrius DSMZ 21285 gewählt ist, gelingt es, insbesondere kompetitiv Brachyspiren hyodysenteriae im Verdauungstrakt von Tieren, insbesondere von Ferkeln zu hemmen. Mit Rücksicht auf die Tatsache, daß üblicherweise in Ferkelmastbetrieben nicht nur Infektionen durch Brachyspiren hyodysenteriae auftreten, sondern insbesondere synergistische Infektionen zwischen Brachyspiren hyodysenteriae und anderen Erregern auftreten, hat sich der Einsatz von insbesondere auch Lactobacillus salivarius DSMZ 21286 und Lactobacillus sobrius DSMZ 21285 besonders bewährt, da diese beiden Gruppen neben der Wirksamkeit gegenüber Brachyspiren hyodysenteriae auch noch bedeutend höhere Wirksamkeit gegenüber E. coli Bakterien, welche, wie bereits ausgeführt, normalerweise synergistisch mit Brachyspiren auftreten, aufweisen, so daß lediglich durch Verabreichung von wenigstens einem Mikroorganismus aus der oben genannten Gruppe eine deutliche Herabsetzung der Krankheitshäufigkeiten und insbesondere die Schwere der Erkrankungen und vor allem die Morbidität von Jungtieren deutlich abzusenken, wobei die gewählten Stämme aus der oben genannten Gruppe von Mikroorganismen sich als besonders effizient gegenüber Brachyspiren und E. coli erwiesen haben sowie eine verbesserte Futterverwertung in Ferkelmastbetrieben beobachtet werden kann.

Indem, wie dies einer bevorzugten Weiterbildung des probiotischen Futtermittel- und/oder Trinkwasserzusatzes gemäß der vorliegenden Erfindung entspricht, zwei oder mehrere Mikroorganismen eingesetzt sind und wenigstens einer der zusätzlich eingesetzten Mikroorganismen aus der Gruppe Enterococcus faecium, Lactobacillus reuteri oder Bacillus subtilis gewählt ist, kann durch Anwendung des sogenannten Mehrstammkonzepts, bei welchem anzunehmen ist, daß unterschiedliche, probiotische Stämme auch unterschiedliche Wirkmechanismen nutzen und somit der kombinierte Einsatz von verschiedenen Bakterienstämmen eine effektive synergistische Nutzung der Mehrwerteigenschaften bewirkt, neben der Tatsache, daß pathogene Keime des Stammes Brachyspira hyodysenteriae gehemmt werden, auch eine weitere Gruppe von Mikroorganismen mit Sicherheit gehemmt werden, wie insbesondere E. coli Bakterien, welche aufgrund ihres häufigen Vorkommens bei wirtschaftlichen Verlusten durch Todesfälle eine besonders große Rolle spielen. Neben E. coli Bakterien können durch Verabreichung von wenigstens einem zusätzlich eingesetzten Mikroorganismus aus der oben genannten Gruppe vor allem auch Clostridium perfringens, Campylobacteri jejuni und Salmonella choleraesuis gehemmt werden.

Besonders effiziente Wirkungen können gemäß der vorliegenden Erfindung dadurch erzielt werden, daß bevorzugt der zusätzliche eingesetzte Mikroorganismus aus der Gruppe Enterococcus faecium DSMZ 3530, DSMZ 19764 oder DSMZ 16211, Lactobacillus reuteri DSMZ 21288 oder Bacillus subtilis DSMZ 21287 gewählt ist. Indem zusätzlich ein Mikroorganismus, gewählt aus der oben genannten Gruppe eingesetzt wird, gelingt es, neben der Hemmung von Brachyspiren hyodysenteriae auch eine große Gruppe von pathogenen E. coli Bakterien zu hemmen. Pathogene E. coli Bakterien verursachen bei Tieren insbesondere nach dem Absetzen einen starken, wäßrigen Durchfall, bei welchem Tiere häufig wegen Entkräftung oder Dehydration sterben. Auch treten bei Jungtieren, insbesondere Schweinen, pathogene Serotypen von E. coli auf, welche Ödemkrankheiten verursachen, wodurch dann die Mortalität von Jungtieren noch weiter ansteigt. Durch Verabreichung einer Kombination von Mikroorganismen, welche kompetitiv sowohl Brachyspiren als auch pathogene E. coli Bakterien und andere in Därmen von Jungtieren vorhandene pathogene Keime hemmen, gelingt es nicht nur die Mortalität von Jungtieren deutlich abzusenken, sondern auch verbesserte Zuchtergebnisse und insbesondere verbesserte Gewichtszunahmen in kürzerer Zeit zu erreichen.

Auch durch Einsatz von Bifidobacterium animalis DSMZ 16284 in Kombination mit beispielsweise Bifidobacterium thermophilium DSMZ 19765 und/oder Lactobacillus salivarius DSMZ 21286 können eine Mehrzahl von pathogenen Darmbakterien in Jungtieren gehemmt werden.

Indem, wie dies einer bevorzugten Weiterbildung der vorliegenden Erfindung entspricht, ausschließlich Mikroorganismen ohne übertragbare Antibiotikaresistenzen gegen Erythromycin oder Tetracyclin eingesetzt sind, kann darüber hinaus sichergestellt werden, daß, sollte es im Zuge der Aufzucht von Jungtieren erforderlich werden, Antibiotika in irgendeiner Weise zu verabreichen, diese auch entsprechende, nicht abgeschwächte Wirkungen zeigen.

Indem, wie dies einer bevorzugten Weiterbildung der vorliegenden Erfindung entspricht, in dem Futtermittel- und/oder Trinkwasserzusatz zusätzlich als Träger einsetzbare Nährsubstanzen und/oder prebiotische Substanzen, gewählt aus Oligosacchariden, Inulinen, Lactitol, Lactosucrose, Lactulose, Pyrodextrinen, Hefen und Hefederivaten, Vitaminen, insbesondere Vitamin E, enthalten sind, werden die Lebensbedingungen der eingesetzten Mikroorganismen im Verdauungstrakt deutlich verbessert und es wird insbesondere dafür Sorge getragen, daß sich der bzw. die eingesetzte (n) Mikroorganismus (-organismen) im Verdauungstrakt rasch und zuverlässig vermehren kann bzw. können und gleichzeitig jedoch die Vermehrung von pathogenen Keimen durch "competitive exclusion" hintangehalten bzw. verhindert wird. Aufgrund der eingesetzten Nährsubstanzen und/oder prebiotischen Substanzen wird ein Wachstumsvorteil für den bzw. die eingesetzten Mikroorganismus (-organismen) gegenüber pathogenen Keimen geschaffen.

Indem, wie dies einer bevorzugten Weiterbildung der vorliegenden Erfindung entspricht, ein weiterer Träger, gewählt aus Zeolithen, Calciumkarbonat, Magnesiumkarbonat, Trehalose, Chitosan, Aluminiumsilikaten, insbesondere granulierten Bentoniten, Stärke, Magermilchpulver, Süßmolkenpulver, Maltodextrine, Lactose, Inulin, Dextrosen, pflanzlichen Ölen, oder ein Lösungsmittel, gewählt aus Wasser oder physiologischer Kochsalzlösung, enthalten ist, kann einerseits eine gleichmäßige Verteilung der Mikroorganismen im Verdauungstrakt erzielt werden und andererseits eine weitere Unterstützung der "competitive exclusion" erzielt werden.

Gemäß einer bevorzugten Weiterbildung hat es sich insbesondere als günstig erwiesen, den erfindungsgemäßen Futtermittel- und/oder Trinkwasserzusatz in suspendierter, pulverförmiger oder verkapselter Form zum Einsatz zu bringen, wodurch je nach gewünschtem Einsatzort, wie beispielsweise Dickdarm, Dünndarm und dgl., eine exakte, gezielte Freisetzung der Wirkung der Mikroorganismen erzielbar ist. Darüber hinaus können selbstverständlich auch Mischformen aus suspendiertem und verkapseltem Futtermittel- und/oder Trinkwasserzusatz zum Einsatz gebracht werden, um die Wirkung über das gesamte Verdauungssystem der damit zu behandelnden Tiere sicherstellen zu können.

Gemäß einer bevorzugten Weiterbildung enthält der Futtermittelzusatz und/oder Trinkwasserzusatz zusätzlich 1% - 99 %, insbesondere 50 % - 95 %, eines Trägers bzw. Naturstoffes. Dadurch, daß zusätzlich ein Träger bzw. Naturstoff enthalten ist, gelingt eine weitere Verdrängung von pathogenen Keimen aus dem Verdauungstrakt, indem das gezielte Wachstum der eingesetzten Mikroorganismen unterstützt bzw. gefördert wird.

Indem, wie dies einer bevorzugten Weiterbildung der Erfindung entspricht, das Futtermittel den Futtermittelzusatz als wäßrige Suspension in einer Menge von 0,01 g/kg - 10 g/kg Futtermittel, insbesondere 0,025 g/kg bis 2,5 g/kg Futtermittel, enthält, wird einerseits sichergestellt, daß der Futtermittelzusatz gemeinsam mit dem Futtermittel problemlos von den Tieren aufgenommen wird und andererseits bereits vor Aufnahme des Futtermittels auf der Oberfläche des Futtermittels vorhandene, pathogene Keime durch den Futtermittelzusatz bekämpft bzw. verdrängt oder abgebaut werden können.

Um eine besonders gute Wirkung des Futtermittelzusatzes auf dem Futtermittel und im Verdauungstrakt der Tiere zu erzielen, weist das Futtermittel den Futtermittelzusatz bevorzugt in einer Menge von 20 g/t bis 20 kg/t, insbesondere 100 g/t bis 2,5 kg/t auf, wodurch einerseits eine ausreichende Menge an Mikroorganismen bereitgestellt ist und andererseits ein sicherer Abbau bzw. eine sichere Verdrängung von pathogenen Keimen aus dem Verdauungstrakt sichergestellt werden kann.

Gemäß einer weiteren Aufgabe zielt die vorliegende Erfindung darauf ab, durch eine gezielte Verwendung von wenigstens einem spezifischen Mikroorganismus einen Futtermittel- bzw. Trinkwasserzusatz herzustellen, der kompetitiv pathogene Keime des Stammes Brachyspira hyodysenteriae im Verdauungstrakt von Tieren hemmt, und weiterhin möglichst viele andere pathogene Keime, wie E. coli ebenfalls kompetitiv hemmt.

Zur Lösung dieser Aufgaben wird gemäß der vorliegenden Erfindung wenigstens ein Mikroorganismus, der aus der Gruppe Bifidobacterium thermophilium, Lactobacillus salivarius und Lactobacillus sobrius gewählt ist, zur Herstellung eines probiotischen Futtermittel- bzw, Trinkwasserzusatzes, zur kompetitiven Hemmung von pathogenen Keimen des Stammes Brachyspira hyodysenteriae im Verdauungstrakt von Tieren verwendet. Durch eine derartige Verwendung gelingt insbesondere, die Herstellung eines probiotischen Futtermittel- bzw. Trinkwasserzusatzes, der die sich in der Tieraufzucht, insbesondere Ferkelaufzucht, als besonders gefährlich erweisenden, pathogenen Keime des Stammes Brachyspira hyodysenteriae kompetitiv im Verdauungstrakt von Tieren hemmt und gleichzeitig kann durch Verabreichung von wenigstens einem Mikroorganismus, gewählt aus der obigen Gruppe, die Futterverwertung gesteigert werden und die Gewichtszunahme im Vergleichszeitraum verbessert werden.

aus der Gruppe Bifidobacterium thermophilium DSMZ 19765, Lactobacillus salivarius DSMZ 21286, Lactobacillus sobrius DSMZ 21285, verwendet wird, wie dies einer bevorzugten Ausführungsform der Erfindung entspricht. Durch Einsatz gezielt ausgewählter Stämme gelingt eine noch bessere Hemmung von Brachyspira hyodysenteriae.

Indem, wie dies einer bevorzugten Weiterbildung der Erfindung entspricht, zwei oder mehrere Mikroorganismen eingesetzt werden und wenigstens einer der zusätzlichen eingesetzten Mikroorganismen aus einer der Gruppe Enterococcus faecium, Lactobacillus reuteri oder Bacillus subtilis gewählt wird, gelingt es neben Brachyspira hyodysenteriae auch insbesondere E. coli, Salmonella choleraesuis, Clostridium perfringens und Campylobacteri jejuni im Verdauungstrakt von Tieren kompetitiv zu hemmen und die schädliche Wirkung dieser Mikroorganismen hintanzuhalten, wobei besonders gute Wirkungen erzielt werden, wenn der zusätzlich eingesetzte Mikroorganismus aus der Gruppe Enterococcus faecium DSMZ 3530, DSMZ 19764 oder DSMZ 16211, Lactobacillus reuteri DSMZ 21288 oder Bacillus subtilis DSMZ 21287 gewählt wird, wie dies einer bevorzugten Ausführungsform der Erfindung entspricht. Gegebenenfalls können auch durch Einsatz von Bifidobacterium animalis DSM 16284 günstige Wirkungen erzielt werden.

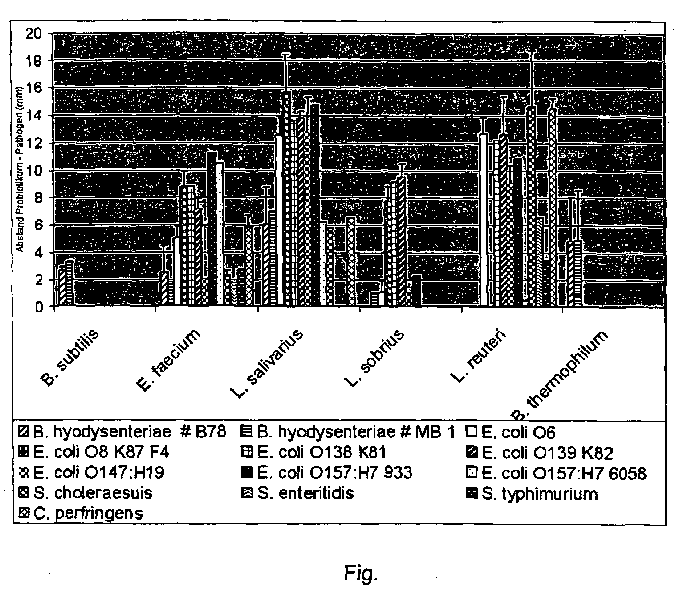

Die Erfindung wird nachfolgend anhand von Ausführungsbeispielen und der Figur näher erläutert, in welchen einerseits die Hemmwirkung der probiotischen Produktstämme gegenüber verschiedenen Krankheitserregern auf Basis der ermittelten Hemmzonen im Agar-Diffusions-Test (mm) und andererseits Fütterungsversuche zur Bestimmung der Wirksamkeit von probiotischem Mischfutter unter Einsatz des erfindungsgemäßen Futtermittel- und/oder Trinkwasserzusatzes zur Kontrolle von Brachyspira hyodysenteriae und zur Erhöhung der Leistung von Tieren gemäß Versuchen 2 bis 4 durchgeführt wurden.

### Versuch 1

Hemmwirkung der probiotischen Produktstämme gegenüber verschiedenen Krankheitserregern auf Basis der ermittelten Hemmzone im Agar-Diffusionstest (mm).

Die zu untersuchenden Keime wurden in dem Versuch in einem Spot bzw. punktförmig auf einem Filterplättchen auf AgarPlatten inkubiert und die gesamte Platte wurde in der Folge mit einer Verdünnung des zu untersuchenden, pathogenen Keims mit halbfestem Agar überschichtet. Nach einer für den jeweiligen Keim optimierten Inkubationszeit wurden die Hemmhöfe ausgemessen. Die Ergebnisse sind einerseits tabellarisch in der nachfolgenden Tabelle 1 dargestellt, in welcher der Abstand der Hemmzone (innerer Bereich zu äußerem Bereich) für einerseits die eingesetzten Mikroorganismen und andererseits die untersuchten, pathogenen Keime dargestellt ist. Des weiteren ist das Ergebnis des Versuchs der Hemmwirkung in der Figur in Form eines Blockdiagramms, in welchem auf der Abszisse die eingesetzten Mikroorganismen und auf der Ordinate der Abstand von Probiotikum - Pathogen angezeigt ist, dargestellt, in welchem die Wirkung von einzelnen eingesetzten Mikroorganismen gegenüber pathogenen Keimen dargestellt ist.

Aus diesem Hemmversuch ergibt sich, daß insbesondere Bifidobakterium thermophilum, Lactobacillus salivarius, Enterococcus faecium und Bacillus subtilis eine besonders gute Wirkung gegen Brachyspira hyodysenteriae zeigen und insbesondere Lactobacillus salivarius und Lactobacillus reuteri extrem gute Wirkungen gegen die verschiedensten E. coli Stämme zeigen.

**Tabelle 1**

| | | B. subtilis | E. faecium | L. salivarius | L. sobrius | L. reuteri | B. thermophilum |
|---|---|---|---|---|---|---|---|
| Abstand Hemmzone innere - äußere Grenze | (mm) | AE1* | FASI13b* | LSH* | FACO 13* | S6* | AN2* |
| B. hyodysenteriae | # B78* | 3 | 2,5 | 6,04 | n.a. | 0 | 4,86 |
| B. hyodysenteriae | # MB 1* | 3 | 1,83 | 7 | 1 | 0 | 4,89 |
| E. coli | O6 | 0 | 5 | 12,58333333 | 1 | 12,675 | 0 |
| E. coli | O8 K87 F4* | 0 | 8,75 | 15,66666667 | 7,75 | 0 | 0 |
| E. coli | O138 K81* | 0 | 8,833333333 | 13,58333333 | 9,16666667 | 12,225 | 0 |
| E. coli | O139 K82* | 0 | 7,166666667 | 13,75 | 9,5 | 12,65 | 0 |
| E. coli | O147:H19* | 0 | 2,055555556 | 12,83333333 | 1 | 9,25 | 0 |
| E. coli | O157:H7 933* | 0 | 11,25 | 14,75 | 2,25 | 10,8333333 | 0 |
| E. coli | O157:H7 6058* | 0 | 10,5 | 6,25 | 0 | 0,5 | 0 |
| S. choleraesuis | | 0 | 2,25 | 5,8 | n.a. | 14,625 | 0 |
| S. enteritidis | | 0 | 1,75 | n.a. | n.a. | 6,58333333 | 0 |
| S. typhimurium | | | 2,75 | n.a. | n.a. | 3,41666667 | 0 |
| C. perfringens | | | 5,833333333 | 6,5 | n.a. | 14,5 | 0 |
| Standardabweichung | | AE1* | FASI13b* | LSH* | FACO 13* | S6* | AN2* |
| E. hyodysenteriae | # B78* | 0,265165043 | 1,9737865 | 2,74481B664 | n.a. | n.a. | 3,142814026 |
| B. hyodysenteriae | # MB 1* | | | | | | 3,657667745 |
| E. coli | O6 | 0 | 0,866025404 | 1,376892637 | 0,90138782 | 1,13069546 | 0 |
| E. coli | O8 K87 F4* | 0 | 1,089724736 | 2,753785274 | 1,040833 | | 0 |
| Abstand Hemmzone innere - äußere Grenze | (mm) | AE1* | FASI13b* | LSH* | FACO 13* | S6* | AN2* |
| E. coli | O138 K81* | 0 | 0,763762616 | 0,5204165 | 1 | 2,70660201 | 0 |
| E. coli | O139 K82* | 0 | 1,842778699 | 2,046338193 | 1,52752523 | 1,7328525 | 0 |
| E. coli | O147:H19* | 0 | 0 | 2,466441431 | 0 | 1,14564392 | 0 |
| E. coli | O157:H7 933* | 0 | 4,518480571 | 1,25 | 0,25 | 0,80363756 | 0 |
| E. coli | O157:H7 6058* | 0 | 0,707106781 | 1,952562419 | k.H. | 0 | 0 |
| S. choleraesuis | | 0 | 0,353553391 | n.a. | n.a. | 4.11045415 | 0 |
| S. enteritidis | | 0 | 0,353553391 | n.a. | n.a. | 1,37689264 | 0 |
| S. typhimurium | | | 0,540061725 | n.a. | n.a. | 1,06848803 | 0 |
| C. perfringens | | | 0,877971146 | | n.a. | 0,70710678 | 0 |

| | | | | | | | |
|---|---|---|---|---|---|---|---|
| * ... Stammcode n.a.... nicht anwendbar k.H.... keine Hemmung H.... erkennbare Hemmung, aber nicht auswertbar 0,00 ... Hemmhof gleich groß wie Kolonie | | | | | | | |

### Versuch 2

Hemmwirkung ausgewählter Isolate gegenüber Brachyspira hyodysenteriae auf Basis der ermittelten Hemmzone im Agardiffusionstest.

Die zu untersuchenden Keime wurden in dem Versuch in einem Spot bzw. punktförmig auf einem Filterblättchen auf Agarplatten inkubiert und die gesamte Platte wurde in der Folge mit einer Verdünnung des zu untersuchenden, pathogenen Keims mit halbfesten Agar überschichtet. Nach einer für den jeweiligen Keim optimierten Inkubationszeit wurden die Hemmhöfe ausgemessen. Die Ergebnisse sind tabellarisch in der nachfolgenden Tabelle 2 dargestellt, in welcher einerseits die Fähigkeit Brachyspira hyodysenteriae zu hemmen gezeigt ist und andererseits untersucht wurde, ob die entsprechenden Stämme eine relevante Antibiotikaresistenz aufweisen.

Aus diesem Hemmversuch ergibt sich, daß insbesondere Lactobacillus salivarius DSMZ 21286 und Bifidobacterium thermophilium DSMZ 19765 eine sehr gute Wirkung zeigen und keine relevante Antibiotikaresistenz zeigen. Des weiteren konnte nachgewiesen werden, daß mehrere Stämme der Spezies Enteroccocus faecium, Lactobacillus salivarius DSMZ 16351, Lactobacillus sobrius DSMZ 21286, Bacillus subtilis DSMZ 21287 eine deutliche Hemmwirkung sowie mit Ausnahme von Bacillus subtilis DSMZ 21287 keine Antibiotikaresistenzen zeigten.

| | Stammbezeichnung | DSMZ Hinter-legungs nummer | Spezies | Hemmung B. Hyodysenteriae | Relevante Antibiotikaresistenz |
|---|---|---|---|---|---|
| | FASI 13b | 19764 | Enterococcus faecium | + | - |
| | IMB 52 | 3530 | Enterococcus faecium | + | - |
| | C5JeMMan4 | 16211 | Enterococcus faecium | + | - |
| | C5EmB12 | - - | *Enterococcus* faecium | + | - |
| | C5EmB17 | - - | *Enterococcus faecium* | - | - |
| | BRE 1b | - - - | *Enterococcus* faecium | - | - |
| | BAR | - - | Enterococcus faecium | - | - |
| | C5CaMMan1 | 16351 | Lactobacillus salivarius | + | - |
| | FACO 27 | - - | *Lactobacillus salivarius* | - | - |
| | LSH | 21286 | Lactobacillus salivarius | ++ | - |
| | SX | 21288 | Lactobacillus reuteri | - | +/- |
| | S6 | 21443 | Lactobacillus reuteri | - | - |
| | SR | - - | *Lactobacillus* reuteri | - | - |
| | FACO 13 | 21285 | *Lactobacillus sobrius* | + | - |
| | AE1 | 21287 | Bacillus subtilis | + | + |
| | C5II(2P)Bian7/2 | 16284 | *Bifidobacterium* animalis | + | - |
| | AN9 1b | - - | Bifidobacterium thermophilum | - | - |
| | AN 2 | 19765 | Bifidobacterium thermophilium | ++ | - |

### Versuch 3

Fütterungsversuch zur Bestimmung der Wirksamkeit von probiotischen Mischungen zur Kontrolle von Brachyspira hyodysenteriae und Escherichia coli und Erhöhung der Leistung

Der Versuch wurde in einem Versuchsstall mit 14 Buchten für jeweils 10 Tiere durchgeführt. Der Stall war ausgestattet mit Spaltenboden, Schalentränkern und einem computer-gesteuerten Fütterungssystem. Die Automaten sind entlang der Buchtenwände angeordnet. Das Stallklima wurde täglich automatisch aufgezeichnet und die Temperatur nach den Standardempfehlungen zur Ferkelaufzucht eingestellt. 133 gemischt-geschlechtliche Absetzferkel im Alter von etwa 4 Wochen mit einem durchschnittlichen Einstellgewicht von 8,11 kg wurden für diesen Versuch eingesetzt. Jedes Ferkel wurde mit einer Ohrmarke versehen, einzeln gewogen und insgesamt 13 Tiere aus dem Versuch ausgeschieden. Die verbleibenden 120 Ferkel wurden auf 12 Buchten zufällig verteilt. Alle Ferkel entstammten dem österreichischen Zuchtprogramm ÖHYB, d.h. (Edelschwein x Landrasse) x Pietrain.

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte 2-phasig: Absetzphase Tag 1 - 14, Aufzuchtphase Tag 15-56. Das Versuchsfutter wurde buchtenindividuell über die eine Fütterungsanlage gemischt und je nach Anzahl der Ferkel, Gewichtsentwicklung und Futterverzehr 2 x täglich trocken zugeteilt. Wasser stand zur Aufnahme ad libitum zur Verfügung.

Der Versuch wurde mit 4 Gruppen zu je 3 Wiederholungen durchgeführt.

Die Gruppen waren:
A - Kontrolle ohne Zusatz
B - Bifidobacterium thermophilium DSMZ 19765;
C - Enterococcus faecium DSMZ 19764, Lactobacillus salivarius DSMZ 21286 und Bifidobacterium thermophilium DSMZ 19765
D - Enterococcus faecium DSMZ 19764, Lactobacillus sobrius DSMZ 21285.

Die Gesamtkeimzahlen der probiotischen Mischungen betrugen jeweils ca. 2,5 x 10⁵ KBE/kg Futter.

Um die Streuung innerhalb der Gruppen möglichst gering zu halten, wurden die Merkmale Wurfgeschwister und Anfangsgewicht bei der Gruppeneinteilung berücksichtigt. Von jeder Sau wurden möglichst viele gleichwertige Vierlinge gebildet und gleichmäßig auf die Gruppen bzw. Buchten aufgeteilt.

Die Tiere wurden zu Versuchsbeginn, an Tag 14, 42 und zu Versuchsende einzeln gewogen und daraus die tägliche Zunahme in den einzelnen Versuchsabschnitten ermittelt. Die zugeteilte Menge Futter wurde täglich buchtenindividuell über den Computer erfaßt. Auch die Klimadaten wurden täglich über den Computer aufgezeichnet. Die Tiere wurden mindestens 2 x täglich kontrolliert und alle Vorkommnisse im Stallbuch aufgezeichnet.

**Tabelle 3: Lebendgewicht (kg) und Ausfälle (Anzahl Tiere)**

| | Gruppe A (Kontrolle) | Gruppe B | Gruppe C | Gruppe D |
|---|---|---|---|---|
| Anzahl Tiere | 30 | 30 | 30 | 30 |
| Anfangsgewicht | 8,32 | 8,36 | 8,43 | 8,38 |
| Gewicht Tag 14 | 10,70 | 10,90 | 11,12 | 10,98 |
| Gewicht Tag 42 | 26,30 | 26,75 | 26,76 | 26,62 |
| Gewicht Tag 56 | 35,00 | 35,42 | 36,78 | 36,03 |
| Ausfälle | 2 | - - | - - | - - |

**Tabelle 4: Futterverwertung (FCR; kg/kg)**

| | Gruppe A (Kontrolle) | Gruppe B | Gruppe C | Gruppe D |
|---|---|---|---|---|
| Tag 1 - 56 | 1,75 | 1,70 | 1,72 | 1,71 |

Aus diesem Versuch ergibt sich, daß sämtliche Versuchsgruppen, welchen Mikroorganismen gemäß der vorliegenden Erfindung verabreicht wurden, 56 Tage ohne Ausfälle gezüchtet werden konnten, daß ihre Gewichtszunahme deutlich über jener der Vergleichsgruppe lag und daß darüber hinaus jene Tiere, welchen Bifidobacterium thermophilium DSMZ 19765 verabreicht wurde, die beste Futterverwertung zeigten.

Die fäkalen Ausscheidungen der Tiere aller Versuchsgruppen wurden einer Analyse auf Brachyspira hyodysenteriae und E. coli unterzogen. Es konnte festgestellt werden, daß die Konzentration der Brachyspiren in den Gruppen mit probiotischen Zusätzen im Vergleich zu der Kontrollgruppe deutlich reduziert war und in der Gruppe, welcher eine Mischung aus drei probiotischen Stämmen zugefüttert wurde (Versuchsgruppe C), lag die Konzentration von Brachyspiren unter der nachweisbaren Grenze. Im Fall von E. coli gab es in allen Versuchsgruppen eine deutliche Reduktion der E. coli Keimzahlen; sie war in den Versuchsgruppen um mindestens zwei Zehnerpotenzen geringer als in der Kontrollgruppe.

### Versuch 4

Fütterungsversuch zur Bestimmung der Wirksamkeit von probiotischen Mischungen zur Kontrolle von Brachyspira hyodysenteriae und Erhöhung der Leistung

Der Versuch wurde in einem Versuchsstall mit 24 Buchten für jeweils 10 Tiere durchgeführt. Der Stall war ausgestattet mit Spaltenboden, Schalentränkern und einem computer-gesteuerten Fütterungssystem. Die Automaten sind entlang der Buchtenwände angeordnet. Das Stallklima wurde täglich automatisch aufgezeichnet und die Temperatur nach den Standardempfehlungen zur Ferkelzucht eingestellt.

240 gemischt-geschlechtliche Absetzferkel im Alter von etwa 4 Wochen mit einem durchschnittlichen Einstellgewicht von 8,02 kg wurden für diesen Versuch eingesetzt. Jedes Ferkel wurde mit einer Ohrmarke versehen und einzeln gewogen und auf 24 Buchten zufällig verteilt. Alle Ferkel entstammen dem österreichischen Zuchtprogramm ÖHYB, d.h. (Edelschwein x Landrasse) x Pietrain.

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte in 2 Phasen: Absetzphase Tag 1 - 14, Aufzuchtphase Tag 15 - 56. Wasser stand zur Aufnahme ad libitum zur Verfügung.

Der Versuch wurde mit 7 Gruppen zu je 3 Wiederholungen durchgeführt.

Die Gruppen waren:
A - Kontrolle ohne Zusatz
B - Lactobacillus salivarius DSMZ 21286
C - Lactobacillus sobrius DSMZ 21284
D - Bifidobacterium animalis DSMZ 16351
E - Bifidobacterium thermophilum DSMZ 19765, Enterococcus faecium DSMZ 3530
F - Lactobacillus reuteri DSMZ 21288, Bifidobacterium thermophilum DSMZ 19765, Lactobacillus salivarius DSMZ 21286
G - Bifidobacterium thermophilum DSMZ 19765, Enterococcus faecium DSMZ 19764, Bacillus subtilis DSMZ 21287

Die Gesamtkeimzahlen der probiotischen Mischungen betrugen jeweils ca. 1 x 10⁵ KBE/kg Futter.

### Ergebnisse:

| | A | B | C | D | E | F | G |
|---|---|---|---|---|---|---|---|
| Anzahl der Tiere | 30 | 30 | 30 | 30 | 30 | 30 | 30 |
| Anfangsgewicht | 8,21 | 8,07 | 8,33 | 8,01 | 8,12 | 7,95 | 8,09 |
| Gewicht Tag 14 | 10,55 | 10,88 | 10,72 | 10,79 | 11,01 | 10,95 | 10,63 |
| Gewicht Tag 56 | 33,62 | 34,68 | 34,17 | 34,56 | 35,72 | 35,23 | 35,16 |
| Ausfälle | 1 | - | - | - | - | - | - |

Neben der signifikanten Verbesserung der Leistungsdaten (Gewichtsentwicklung) konnte mittels PCR Analytik festgestellt werden, daß im Kot der Versuchgruppen keine Brachyspiren nachgewiesen wurden, während die Kontrollgruppe positiv auf Brachyspiren getestet wurde.

Aus diesen Versuchsergebnissen kann geschlossen werden, daß jene Tiere, welchen probiotische Zusätze verabreicht wurden, die aus lediglich einem einzigen probiotischen Stamm bestanden, deutlich höhere Gewichtszunahmen zeigten als die Kontroll- bzw. Vergleichsgruppe, und daß bei Verabreichung von Mischungen von mehreren probiotischen Stämmen weitere Gewichtserhöhungen erzielt werden können.

### Versuch 5

Fütterungsversuch zur Bestimmung der Wirksamkeit von probiotischen Mischungen zur Kontrolle von Brachyspira hyodysenteriae und Erhöhung der Leistung

Der Versuch wurde in einem Versuchsstall mit 14 Buchten für jeweils 10 Tiere durchgeführt. Der Stall war ausgestattet mit Spaltenboden, Schalentränkern und einem computer-gesteuerten Fütterungssystem. Die Automaten sind entlang der Buchtenwände angeordnet. Das Stallklima wurde täglich automatisch aufgezeichnet und die Temperatur nach den Standardempfehlungen zur Ferkelaufzucht eingestellt.

133 gemischt-geschlechtliche Absetzferkel im Alter von etwa 4 Wochen mit einem durchschnittlichen Einstellgewicht von 8,11 kg wurden für diesen Versuch eingesetzt. Jedes Ferkel wurde mit einer Ohrmarke versehen, einzeln gewogen und insgesamt 13 Tiere aus dem Versuch ausgeschieden. Die verbleibenden 120 Ferkel wurden auf 12 Buchten zufällig verteilt. Alle Ferkel entstammten dem österreichischen Zuchtprogramm ÖHYB, d.h. (Edelschwein x Landrasse) x Pietrain.

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte 2-phasig: Absetzphase Tag 1 - 14, Aufzuchtphase Tag 15-56. Das Versuchsfutter wurde buchtenindividuell über die eine Fütterungsanlage gemischt und je nach Anzahl der Ferkel, Gewichtsentwicklung und Futterverzehr 2 x täglich trocken zugeteilt. Wasser stand zur Aufnahme ad libitum zur Verfügung.

Der Versuch wurde mit 4 Gruppen zu je 3 Wiederholungen durchgeführt.

Die Gruppen waren:
A - Kontrolle ohne Zusatz
B - Enterococcus faecium DSMZ 16211 und Lactobacillus salivarius DSMZ 21286 ;
C - Enterococcus faecium DSMZ 3530, Lactobacillus salivarius DSMZ 21286 und Bifidobacterium thermophilium DSMZ 19765
D - Enterococcus faecium DSMZ 19764

Die Gesamtkeimzahlen betrugen jeweils ca. 5 x 10⁵ KBE/kg Futter.

Um die Streuung innerhalb der Gruppen möglichst gering zu halten, wurden die Merkmale Wurfgeschwister und Anfangsgewicht bei der Gruppeneinteilung berücksichtigt. Von jeder Sau wurden möglichst viele gleichwertige Vierlinge gebildet und gleichmäßig auf die Gruppen bzw. Buchten aufgeteilt.

Die Tiere wurden zu Versuchsbeginn, an Tag 14, 42 und zu Versuchsende einzeln gewogen und daraus die tägliche Zunahme in den einzelnen Versuchsabschnitten ermittelt. Die zugeteilte Menge Futter wurde täglich buchtenindividuell über den Computer erfaßt. Auch die Klimadaten wurden täglich über den Computer aufgezeichnet. Die Tiere wurden mindestens 2 x täglich kontrolliert und alle Vorkommnisse im Stallbuch aufgezeichnet.

**Tabelle 5: Lebendgewicht (kg) und Ausfälle (Anzahl Tiere)**

| | Gruppe A (Kontrolle) | Gruppe B | Gruppe C | Gruppe D |
|---|---|---|---|---|
| Anzahl Tiere | 30 | 30 | 30 | 30 |
| Anfangsgewicht | 8,32 | 8,36 | 8,43 | 8,38 |
| Gewicht Tag 14 | 10,70 | 10,90 | 11,02 | 10,78 |
| Gewicht Tag 42 | 26,30 | 26,75 | 26,66 | 26,62 |
| Gewicht Tag 56 | 35,00 | 35,42 | 36,51 | 35,03 |
| Ausfälle | 2 | - - | - - | - - |

**Tabelle 6: Tägliche Zunahme (g)**

| | Gruppe A (Kontrolle) | Gruppe B | Gruppe C | Gruppe D |
|---|---|---|---|---|
| Tag 1 - 14 | 170 | 181 | 185 | 172 |
| Tag 15 - 42 | 545 | 566 | 559 | 566 |
| Tag 1 - 42 | 423 | 438 | 434 | 434 |
| Tag 15 - 56 | 571 | 584 | 607 | 577 |
| Tag 43 - 56 | 622 | 619 | 704 | 600 |
| Tag 1 - 56 | 473 | 483 | 501 | 476 |

**Tabelle 7: Futterverwertung (FCR; kg/kg)**

| | Gruppe A (Kontrolle) | Gruppe B | Gruppe C | Gruppe D |
|---|---|---|---|---|
| Tag 1 - 14 | 1,34 | 1,26 | 1,28 | 1,28 |
| Tag 15 - 42 | 1,71 | 1,65 | 1,66 | 1,65 |
| Tag 1 - 42 | 1,66 | 1,59 | 1,61 | 1,60 |
| Tag 15 - 56 | 1,79 | 1,75 | 1,76 | 1,76 |
| Tag 43 - 56 | 1,94 | 1,93 | 1,92 | 1,96 |
| Tag 1 - 56 | 1,75 | 1,70 | 1,72 | 1,71 |

Die mit der Mischung aus den 3 probiotischen Stämmen gefütterten Tiere wiesen bereits nach 14 Tagen tendenziell verbesserte Werte auf. Im weiteren Versuchsverlauf konnte der Trend der ersten 14 Tage auch weiterhin beobachtet werden. Alle Versuchsgruppen lagen über der Kontrolle, die Gruppe mit den 2 Stämmen erreichte ein um knapp 0,5 kg höheres Endgewicht, in der Gruppe mit den 3 probiotischen Mikroorganismen lag der Unterschied zur Kontrolle bei + 1,5 kg. Auch die Gruppe mit Enterococcus faecium lag im Vergleich zu Kontrolle etwas höher.

Hinsichtlich der täglichen Zunahmen konnte Ähnliches beobachtet werden: durch den Zusatz des 2-Stamm-Gemisches konnte eine Leistungssteigerung von 473 auf 478 g erreicht werden, in der Gruppe mit den 3 Stämmen auf 501 g. Speziell in der letzten Versuchsphase (Tag 43 - 56) konnte in der Gruppe mit den 3 Stämmen eine signifikante Steigerung (+ 104 g) der täglichen Zunahme im Vergleich zur Vergleichsgruppe erreicht werden. Im Vergleich zur Kontrolle und der Gruppe mit den 2 Stämmen betrug die Leistungssteigerung 82 bis 85 g. Die einzigen beiden Ausfälle im Versuch waren in der Kontrollgruppe zu verzeichnen. Hinsichtlich Futterverwertung konnte in allen Versuchsgruppen eine Verbesserung im Vergleich zur Kontrolle beobachtet werden.

Die fäkalen Ausscheidungen von Tieren aller Versuchsgruppen wurden einer Analyse auf Brachyspira hyodysenteriae unterzogen. Es konnte festgestellt werden, daß die Reduktion von Brachyspira in den Gruppen mit probiotischen Zusätzen im Vergleich zu der Kontrollgruppe deutlich reduziert war und in der Gruppe mit den 3 probiotischen Stämmen lag die Konzentration von Brachyspiren unter der Nachweisgrenze.

## Patentansprüche

1. Probiotischer Futtermittel- und/oder Trinkwasserzusatz, enthaltend wenigstens einen Mikroorganismus zur Anwendung zur kompetitive Hemmung von pathogenen Keimen des Stammes Brachyspira hyodysenteriae im Verdauungstrakt von Tieren, dadurch gelcennzeichnet, daß der wenigstens eine Mikroorganismus aus der Gruppe Bifidobacterium thermophilium DSMZ 19765, Lactobacillus salivarius DSMZ 21286, Lactobacillus sobrius DSMZ 21285 gewählt ist.

2. Probiotischer Futtermittel- und/oder Trinkwasserzusatz zur Anwendung nach Anspruch 1, **dadurch gekennzeichnet, daß** zwei oder mehrere Mikroorganismen eingesetzt sind und daß wenigstens einer der zusätzlich eingesetzten Mikroorganismen aus der Gruppe Enterococcus faecium, Lactobacillus reuteri oder Bacillus subtilis gewählt ist.

3. Probiotischer Futtermittel- und/oder Trinkwasserzusatz zur Anwendung nach Anspruch 1 oder 2, **dadurch gekennzeichnet, daß** der zusätzlichen eingesetzte Mikroorganismus aus der Gruppe Enterococcus faecium DSMZ 3530, DSMZ 19764 oder DSMZ 16211, Lactobacillus reuteri DSMZ 21288 oder Bacillus subtilis DSMZ 21287 gewählt ist.

4. Probiotischer Futtermittel- und/oder Trinkwasserzusatz zur Anwendung nach Anspruch 3, **dadurch gekennzeichnet**, das ausschließlich Mikroorganismen ohne übertragbare Antibiotiokaresistenzen gegen Erythromycin oder Tetracyclin eingesetzt sind.

5. Probiotischer Futtermittel- und/oder Trinkwasserzusatz zur Anwendung nach einem der Ansprüche 1 bis 4, **dadurch gekennzeichnet, daß** zusätzlich als Träger einsetzbare Nährsubstanzen und/oder prebiotische Substanzen, gewählt aus Oligosacchariden, Inulinen, Lactitol, Lactosucrose, Lactulose, Pyrodextrinen, Hefen und Hefederivaten, Vitaminen, insbesondere Vitamin E, enthalten sind.

6. Probiotischer Futtermittel- und/oder Trinkwasserzusatz zur Anwendung nach einem der Ansprüche 1 bis 5, **dadurch gekennzeichnet, daß** ein weiterer Träger, gewählt aus Zeolithen, Calciumkarbonat, Magnesiumkarbonat, Trehalose, Chitosan, Aluminiumsilikaten, insbesondere granulierten Bentoniten, Stärke, magermilchpulver, Süßmolkenpulver, Maltodextrine, Lactose, Inulin, Dextrosen, pflanzlichen Ölen, oder ein Lösungsmittel, gewählt aus Wasser oder physiologischer Kochsalzlösung, enthalten ist.

7. Probiotischer Futtermittel- und/oder Trinkwasserzusatz zur Anwendung nach einem der Ansprüche 1 bis 6, dadurch gekenntzeichnet, daß die Mikroorganismen in suspendierter, pulverförmiger oder verkapselter Form enthalten sind.

8. Probiotischer Futtermittel- und/oder Trinkwasserzusatz zur Anwendung nach einem der Ansprüche 1 bis 7, **dadurch gekennzeichnet, daß** der Futtermittelzusatz zusätzlich 1 % - 99 %, insbesondere 50 % - 95 %, eines Trägers bzw. Naturstoffes enthält.

9. Futtermittel, enthaltend einen probiotischen Futtermittel- und/oder Trinkwasserzusatz zur Anwendung nach einem der Ansprüche 1 bis 8, dadurch gekenntzeichnet, daß das Futtermittel den Futtermittelzusatz als wäßrige Suspension in einer Menge von 0,01 g/kg - 10 g/kg, insbesondere 0,025 g/kg bis 2,5 g/kg, enthält.

10. Futtermittel, enthaltend einen probiotischen Futtermittel- und/oder Trinkwasserzusatz zur Anwendung nach einem der Ansprüche 1 bis 8, **dadurch gekennzeichnet, daß** das Futtermittel den Futtermittelzusatz in einer Menge von 20 g/t bis 20 kg /t, insbesondere 100 g/t bis 2,5 kg/t, aufweist.

11. Verwendung von wenigstens einem Mikroorganismus der aus der Gruppe Bifidobacterium thermophilium, Lactobacillus Salivarius und Lactobacillus sobrius gewählt ist, zur Herstellung eines probiotischen Futtermittel- bzw. Trinkwasserzusatzes zur kompetitiven Hemmung pathogener Keime des Stammes Brachyspira hyodysenteriae im Verdauungstrakt von Tieren.

12. Verwendung nach Anspruch 11, **dadurch gekennzeichnet, daß** wenigstens ein Mikroorganismus, gewählt aus der Gruppe Bifidobacterium thermophilium DSMZ 19765, Lactobacillus salivarius DSMZ 21286, Lactobacillus sobrius DSMZ 21285, eingesetzt wird.

13. Verwendung nach Anspruch 11 oder 12, **dadurch gekennzeichnet**, das zwei oder mehrere Mikroorganismen eingesetzt werden und daß wenigstens einer der zusätzlichen eingesetzten Mikroorganismen aus einer der Gruppe Enterococcus faecium, Lactobacillus reuteri oder Bacillus subtilis gewählt wird.

14. Verwendung nach einem der Ansprüche 11, 12 oder 13, **dadurch gekennzeichnet, daß** der zusätzlich eingesetzte Mikroorganismus aus der Gruppe Enterococcus faecium DSMZ 3530, DSMZ 19764 oder DSMZ 16211, Lactobacillus reuteri DSMZ 21288 oder Bacillus subtilis DSMZ 21287 gewählt wird.

## Claims

1. A probiotic feed and/or drinking water additive containing at least one microorganism for application to a competitive inhibition of pathogenic germs of the Brachyspira hyodysenteriae strain the digestive tract of animals, **characterized in that** the at least one microorganism is selected from group consisting of Bifidobacterium thermophilium DSMZ 19765, Lactobacillus salivarius DSMZ 21286, Lactobacillus sobrius DSMZ 21285.

2. A probiotic feed and/or drinking water additive for application according to claim 1, **characterized in that** two or more microorganisms are and at least one of the additionally used microorganism is selected from the group consisting of Enterococcus faecium, Lactobacillus reuteri or Bacillus subtilis.

3. A probiotic feed and/or drinking water additive for application according to claim 1 or 2, **characterized in that** the additionally used microorganism is selected from the group consisting of Entereococcus faecium DSMZ 3530, DSMZ 19764 or DMSZ 16211, Lactobacillus reuteri DSMZ 21288 or Bacillus subtilis DSMZ 21287.

4. A probiotic feed and/or drinking water additive for application according to claim 3, **characterized in that** microorganisms without contiguous antibiotic resistances against erythromycin or tetracycline are exclusively used.

5. A probiotic feed and/or drinking water additive for application according to any one of claims 1 to 4, **characterized in that** nutrients and/or prebiotic substances which are usable as carriers and selected from oligosaccharides, inulins, lacititol, lactosucrose, lactulose, pyrodextrines, yeasts and yeast derivatives, vitamins, in particular vitamin E are additionally contained.

6. A probiotic feed and/or drinking water additive for application according to any one of claims 1 to 5, **characterized in that** a further carrier selected from zeolites, calcium carbonate, magnesium carbonate, trehalose, chitosan, aluminium silicates, in particular granulated benotines, starch, powder, sweet-whey powder, maltodextrins, lactose, inulin, dextroses, vegetable oils or a solvent selected from water or physiologic saline solution, is contained.

7. A probiotic feed and/or drinking water additive for application according to any one of claims 1 to 6, **characterized in that** the microorganisms are contained in suspended, powdery or encapsulated form.

8. A probiotic feed and/or drinking water additive for application according to any one of claims 1 to 7, **characterized in that** the feed additive additionally contains 1% - 99%, in particular 50% - 95 %, of a carrier or natural substance.

9. A feed containing a probiotic feed and/or drinking water additive for application according to any one of claims 1 to 8, **characterized in that** the feed contains the feed additive as an aqueous suspension in an amount of 0,01 g/kg to 10 g/kg feed, in particular 0,025 g/kg to 2,5 g/kg feed.

10. A feed containing a probiotic feed and/or drinking water additive for application according to any one of claims 1 to 8, **characterized in that** the feed contains the feed additive in an amount of 20 g/t to 20 kg/t, in particular 100 g/t to 2,5 kg/t.

11. The use of at least one microorganism selected from the group consisting of Bifidobacterium thermophilium, Lactobacillus salivarius and Lactobacillus sobrius for the production of a probiotic feed and/or drinking water additive for the competitive inhibition of pathogenic germs of the Brachyspira hyodysenteriae strain in the digestive tract of animals.

12. The use according to claim 11, **characterized in that** at least one microorganism slected from the group consisting of Bifidobacterium thermophilium DSMZ 19765, Lactobacillus salivarius DSMZ 21286, Lactobacillus sobrius DSMZ 21285 is used. 1

13. The use according to claim 11 or 12, **characterized in that** two or more microorganisms are used, and at least one of the additionally used microorganisms is selected from the group consisting of Enterococcus faecium, Lactobacillus reuteri or Bacillus subtilis

14. The use according to any one of claim 11, 12 or 13, **characterized in that** the additionally employed microorganism is selected from the group consisting of Enterococcus faecium DSMZ 3530, DSMZ 19764 or DSMZ 16211, Lactobacillus reuteri DSMZ 21288 or Bacillus subtilis DSMZ 21287.

## Revendications

1. Additif d'aliment pour animaux et/ou additif d'eau potable probiotique(s) contenant au moins un micro-organisme pour l'utilisation dans l'inhibition compétitive de germes pathogènes de la souche *Brachyspira hyodysenteriae* dans les voies digestives d'animaux, **caractérisé en ce que** redit au moins un micro-organisme est choisi dans le groupe comprenant Bifidobacterium *thermophilium* DSMZ 19765, Lactobacillus salivarius DSMZ 21286, *Lactobacillus sobrius* DSMZ 21285.

2. Additif d'aliment pour animaux et/ou additif d'eau potable probiotique(s) pour l'utilisation selon la revendication 1, caractérisé en que l'on utilise deux ou plusieurs microorganismes, et en ce qu'au moins l'un des micro-organismes utilisé de façon supplémentaire est choisi dans le groupe comprenant *Enterococcus faecium, Lactobacillus reuteri* ou *Bacillus subtilis.*

3. Additif d'aliment pour animaux et/ou additif d'eau potable probiotique(s) pour l'utilisation selon la *revendication* 1 ou 2, **caractérisé en ce que** le micro-organisme utilisé de façon complémentaire est choisi dans le groupe comprenant *Enterococcus faecium* DSMZ 3530, DSMZ 19764 ou DSMZ 16211, *La tobacillus reuteri* DSMZ 21288 ou *Bacillus subtilis* DSMZ 21287.

4. Additif d'aliment pour animaux et/ou additif d'eau potable probiotique(s) pour l'utilisation selon la revendication 3, **caractérisé en ce que** l'on utilise exclusivement des micro-organismes ne présentant pas de résistance transmissible aux antibiotique contre l'érythromycine ou la tétracycline.

5. Additif d'aliment pour animaux et/ou additif d'eau potable probiotique(s) pour l'utilisation selon l'une des revendications 1 à 4, **caractérisé en ce que** sont contenues des substances nutritives utilisables en outre comme supports et/ou des substances prébiotiques choisies parmi les oligosaccharides, les inulines, le lactitol, le lactosaccharose, le lactulose, les pyrodextrines, les levures et les dérivés de levure, les vitamines, en particulier la vitamine E.

6. Additif d'aliment pour animaux et/ou additif d'eau potable probiotique(s) pour l'utilisation selon l'une des revendications 1 à 5, **caractérisé en ce qu'**est contenu un autre support choisi parmi les zéolithes, le carbonate de calcium, le carbonate de magnésium, le tréhalose, le chitosan, les silicates d'aluminium, en particulier les bentonites granulées, les amidons, la poudre de lait écrémé, la poudre de lactosérum doux, les malto-dextrines, le lactose, l'inuline, les dextroses, les huiles végétales, ou un solvant choisi parmi l'eau ou une solution saline physiologique.

7. Additif d'aliment pour animaux et/ou additif d'eau potable probiotique(s) pour l'utilisation selon l'une des revendications 1 à 6, **caractérisé en ce que** les micro-organismes sont contenus sous forme de suspension, sous forme de poudre ou sous forme encapsulée.

8. Additif d'aliment pour animaux et/ou additif d'eau potable probiotique(s) pour l'utilisation selon l'une des revendications 1 à 7, **caractérisé en ce que** l'additif d'aliment pour animaux contient en outre 1 % à 99 %, en particulier 50 % à 95 %, d'un support ou d'une substance naturelle.

9. Aliment pour animaux contenant un additif d'aliment pour animaux et/ou additif d'eau potable probiotique(s) pour l'utilisation selon l'une des revendication 1 à 9, **caractérisé en ce que** l'aliment pour animaux contient l'additif d'aliment pour animaux sous la forme d'une suspension aqueuse en une quantité de 0,01 g/kg à 10 g/kg, en particulier de 0,025 g/kg à 2,5 g/kg.

10. Aliment pour animaux contenant additif d'aliment pour animaux et/ou additif d'eau potable probiotique(s) pour l'utilisation selon l'une des revendication 1 à 8, **caractérisé en ce que** l'aliment anomaux présente l'additif d'aliment pour animaux une quantité de 20 g/t à 20 kg/t, en particulier de 100 g/t à 2,5 kg/t.

11. Utilisation d'au moins un micro-organisme qui est choisi dans le groupe comprenait *Bifidobacterium thermophilium, Lactobacillus Salivarius* et *Lactobacillus sobrius,* pour la production d'un additif d'aliment pour animaux ou additif d'eau potable probiotique pour l'inhibition compétitive de germes pathogènes de la souche *Brachyspira hyodysenteriae* dans les voies digestives d'animaux.

12. Utilisation selon la revendication 11, **caractérisée en ce que** l'on utilise au moins un micro-organisme choisi dans le groupe comprenant *Bifidobacterium thermophilium* DSMZ 19765, Lactobacilus *salivarius* DSMZ 21286, *Lactobacillus sobrius* DSMZ 21285.

13. Utilisation selon la revendication 11 ou 12, **caractérisée en ce que** l'on deux ou plusieurs micro-organismes et qu'au moins l'un des micro-organismes utilisé de façon complémentaire est choisi dans le groupe comprenant *Enterococcus faecium, Lactobacillus reuteri* ou *Bacillus subtilis.*

14. Utilisation selon l'une des revendication 11, 12 ou 13, **caractérisée en ce que** le micro-organisme utilisé de façon complémentaire choisi dans le groupe comprenant *Enterococcus faecium* DSMZ 3530, DSMZ 19764 ou DSMZ 16211, *Lactobacillus reuteri* DSMZ 21288 ou *Bacillus subtilis* DSMZ 21287.
